# EUROPEAN PATENT APPLICATION

(11) **EP 4 830 759 A1**
(43) Date of publication of application: **28.10.2026**
(21) Application number: 25172312.8
(22) Date of filing: 24.04.2025
(51) Int. Cl.: A61M 39/18, A61M 39/02, A61M 39/20, A61M 5/32

(54) **A MEDICAL REPLACING DEVICE, A MEDICAL REPLACING SYSTEM AND A MEDICAL CONTAINER SYSTEM**

(71) Applicant: Transcutan AB, 411 18 Göteborg (SE)
(72) Inventor: ÖRTENGREN, John, 247 36 Södra Sandby (SE); STOLTZ, Fredrik, 247 60 Veberöd (SE); ERIKSSON, Vincent, 215 84 Malmö (SE); TRÄGÅRDH, Peter, 212 14 Malmö (SE); LARSSON, Filip, 234 38 Lomma (SE)
(74) Representative: Zacco Sweden AB

(57) **Abstract**

A medical replacing device for replacing a used port-engaging attachment engaged with a port connected to a medical tube extending inside a human or animal body, A first portion comprises a first actuator, a removing part and a receiving part. The first actuator causes the removing part to become actuated for removing said used port-engaging attachment from said port and into the receiving part. A second portion comprises a second actuator, a discharging part, a compartment having an opening, and an unused port-engaging attachment stored in the compartment and arranged to be discharged through the opening by the discharging part. A protective element covers said opening to maintain the unused port-engaging attachment in the compartment. Upon receipt of the used port-engaging attachment into the receiving part, the second actuator can cause actuation of the discharging part for discharging the unused port-engaging attachment through the opening and onto said port.

## Description

### TECHNICAL FIELD

The present disclosure relates to a medical replacing device. The present disclosure also relates to a medical replacing system which comprises such a medical replacing device. The present disclosure further relates to a medical container system which comprises such a medical replacing device.

### BACKGROUND ART

In the medical field there are various treatments which involve supplying a liquid to a person's body. One such example is peritoneal dialysis, which involves supplying a liquid to the peritoneum in the person's abdomen. To this end, a portion of a catheter may be operated into the body, while another portion is allowed to extend through the skin to the outside of the body. The portion of the catheter that is outside the body presents an inlet which is normally closed by some sort of attachment, for example, a screw cap. When it is time for treatment, the screw cap is unscrewed to expose the inlet and a container, such as a bag, containing liquid (dialysate) is via a tube connected to the inlet to transfer the liquid into the body. Afterwards the screw cap is once again screwed onto the inlet of the catheter to close it.

Some dialysis treatments may preferably be conducted in hospitals or other medical facilities. However, it is quite common that a person (although being a patient or care recipient) may perform the treatment on his/her own, at home. For some person's the treatment needs to be repeated several times a day. For some persons, in particular elderly persons, the procedure of unscrewing the screw cap, connecting the tube, disconnecting the tube, and then again remounting the screw cap, may be quite a hassle. Not only may the actual manoeuvring, such as gripping the various components, present a challenge, but there may also be a risk of the catheter becoming subjected to unwanted bacteria, dirt or other forms of contamination when performing all the procedural steps, which then may enter the person's body. In particular, the repeated touching of the screw cap each time the procedural steps need to be made may cause an accumulation up of such unwanted bacteria, dirt, etc.

### SUMMARY OF THE INVENTION

An object of the present disclosure is to provide a medical replacing device which at least partly mitigates the drawbacks of the prior art. This object, and other objects which will become apparent in the following discussion, is accomplished by a medical replacing device as defined in accompanying the independent claim. Some non-limiting exemplary embodiments are presented in the dependent claims.

The general inventive concept is based on the realization that by providing a medical replacing device which has dual functionality, namely both a removal functionality and a discharging functionality, dialysis treatment or other liquid-to-body treatments may be facilitated. In particular, the general inventive concept is based on the realization that by provided a medical replacing device which can both remove an old attachment and discharge a new attachment, the prior art repeated touching of an attachment (such as the above exemplified screw cap) can be avoided. Furthermore, a person performing the treatment on his/her own may be relieved from at least some of the previously perceived burden since the medical replacing device will simplify at least some parts of the treatment.

It should be noted that although the background description was discussed with reference to peritoneal dialysis, the present disclosure is not limited to such an implementation. The medical replacing device and other aspects of this disclosure may be implemented for other types of dialysis as well, such as for example hemodialysis. In fact, the medical replacing device and other aspects of this disclosure may be implemented in relation to any other suitable treatments that involve providing liquid into a human or animal body.

According to a first aspect of this disclosure, there is provided a medical replacing device configured for replacing a used port-engaging attachment which is engaged with a port connected to a medical tube that extends inside a human or animal body, wherein the medical replacing device comprises:
- a first portion comprising a first actuator, a removing part and a receiving part, wherein the first actuator is configured to cause the removing part to become actuated for removing said used port-engaging attachment from said port and into the receiving part,
- a second portion comprising a second actuator, a discharging part, a compartment having an opening, and an unused port-engaging attachment stored in the compartment and arranged to be discharged through the opening by the discharging part,
- a protective element which covers said opening of the compartment for maintaining the unused port-engaging attachment in the compartment,
wherein the second actuator is configured to cause the discharging part to become actuated for discharging the unused port-engaging attachment through the opening and onto said port. By having a first portion in which a removing part is activated for removing a used port-engaging attachment and a second portion from which an unused port-engaging attachment is discharged some of the manual handling can be avoided by simply activating the first and the second actuator respectively. Thus, there is no need for a person to grip the attachment (such as a screw cap) by hand and replace it by hand, such actions may instead be achieved by means of the medical replacing device.

As will be discussed in more detail later, in some exemplary embodiments the used port-engaging attachment may be in the form of a lid for covering the port when no liquid is to be transferred into the body. In such cases the unused port-engaging attachment, i.e. the new port-engaging attachment, stored in the medical replacing device may suitably be an adapter which can interconnect a liquid source with the port to provide liquid into the body. Hereby, the procedural steps for establishing liquid communication to the body may be achieved without a person needing to touch any attachment (neither the lid, nor the adapter).

As will also be discussed in more detail later, in other exemplary embodiments the used port-engaging attachment may instead be in the form of an adapter, in which case the unused port-engaging attachment in the medical replacing device may be in the form of a lid. Thus, the procedural steps for covering the port, after completing administration of liquid into the body, do not require any touching of the attachments (neither the adapter that is removed, nor the lid that is placed onto the port).

Suitably, a person may therefore conveniently be provided with stashes of the different embodiments of the medical replacing device, so that the person can pick one medical replacing device for initiating the liquid administration (as in the first exemplary embodiment above), and pick another one after completion of the liquid administration (as in the second exemplary embodiment). Although the discussion of the medical replacing device and its various exemplary embodiments, has so far only mentioned two portions, it should be understood that in still further exemplary embodiments, a medical replacing device may comprise additional portions such that more than one attachment can be removed, and more than one attachment can be discharged from one and the same medical replacing device. In such exemplary embodiments, which will be discussed in more detail later, the same medical replacing device may be used for initiating liquid administration and also used after completion of the liquid administration.

As mentioned above, the medical replacing device is provided with a protective element which covers the opening of the compartment. Hereby, premature discharging of the unused port-engaging attachment is counteracted. The protective element may be in any suitable form that achieves this functionality. It does not need to cover the opening completely, but may, in at least some exemplary embodiments, cover the opening partially as long as it counteracts the unused port-engaging attachment from being prematurely discharged.

In some exemplary embodiments, the protective element may be a hard cover, such as a hard plastic cover. Such a hard cover may, for example, be slidably arranged to cover the opening. In other exemplary embodiments, it may be in the form of a release film, which may be adhesively adhered to an area surrounding the opening of the compartment. In some exemplary embodiments, the protective element and the opening are configured to be separable from each other so as to expose the opening before the unused port-engaging attachment is discharged from the compartment through the opening. Such a separation may for example be achieved by removing the protective element, while maintaining the compartment stationary (e.g. stationary relative to the port), whereas in other examples such a separation may be achieved by keeping the protective element stationary (e.g. stationary relative to the port) and moving another part of the medical replacing device including the second portion and its compartment. In other exemplary embodiments, however, the protective element may be strong enough to keep the unused port-engaging attachment in the compartment before use, but weak enough to become pierced, peeled or otherwise displaced due to the force provided by the discharging part when actuated. For instance, the unused port-engaging attachment may, in some exemplary embodiments, be subjected to a force from the discharging part which is high enough to cause the unused port-engaging attachment to pierce through the protective element similarly to a medical pill/tablet that can be pressed through a blister pack.

As mentioned above, the medical replacing device is provided with a removing part in the first portion and a discharging part in the second portion. Various configurations are conceivable for these parts. For instance, the removing part may, in at least some exemplary embodiments, include a gripping element, such as a hook, claw, etc. In other exemplary embodiments, the removing part may include one or more male elements that are configured to mate with respective female elements on the used port-engaging attachment, or vice versa. In still other exemplary embodiments, the removing part may be configured to remove the attachment in a bayonet-type engagement. Thus, various configurations are conceivable for the removing part. Similarly, corresponding examples may be conceivable for the discharging part.

According to at least one exemplary embodiment, upon receipt of the used port-engaging attachment into the receiving part, the second actuator is configured to cause the discharging part to become actuated for discharging the unused port-engaging attachment through the opening and onto said port. By limiting the capability of the second actuator to cause the discharging part to become actuated as a consequence of the used port-engaging attachment having been received into the receiving part, premature actuation of the discharging part may be avoided. It should, however, be noted that the general inventive concept is not limited to the second actuator only being available for usage based on reception of a used port-engaging attachment in the receiving part. On the contrary, the general inventive concept also envisages that a person may in some situations wish to remove the used port-engaging attachment from the port in other ways, for example manually or by some other device. Therefore, for such cases, the second actuator should be useable irrespective of how the used port-engaging attachment is removed.

According to at least some exemplary embodiments, the medical replacing device may be configured to be aligned with said port in two different alignment positions, wherein
- in a first alignment position the first portion is aligned with said port, wherein in said first alignment position, and with the used port-engaging attachment connected to said port, activation of the first actuator causes the removing part to remove the used port-engaging attachment from the port and into the receiving part, and
- in a second alignment position the second portion is aligned with said port, wherein, in said second alignment position, and without any port-engaging attachment connected to the port, activation of the second actuator causes the discharging part to discharge the unused port-engaging attachment through the opening and onto said port.

By having two different alignment positions for the medical replacing device, a sequential and logical replacement procedure can be achieved. Furthermore, by separating the two alignment positions, the risk of the used port-engaging attachment touching/contaminating unused port-engaging attachment can be reduced. It is conceivable to achieve the actual changing from the first alignment position to the second alignment position in various manners. For instance, the change from the first alignment position to the second alignment position may, in some exemplary embodiments, be in the form of a linear displacement. In other exemplary embodiments, the change from the first alignment position to the second alignment position may be a rotational displacement. In any case, the first portion and the second portion may, in at least some exemplary embodiments, be arranged to be movable relative to a housing of the medical replacing device. For instance, the first portion and the second portion may form part of a displaceable part for displacing the first portion and the second portion relative to the housing. In other exemplary embodiments, it is conceivable to let the entire medical replacing device be movable relative to a housing of a separate device. Thus, in a general sense, in at least some exemplary embodiments the first portion and the second portion of the medical replacing device may be displaceable relative to a housing, said housing being forming part of the medical replacing device or forming part of a separate device. Further down below, an actuating device will be discussed, which may represent such a separate device.

According to at least some exemplary embodiments, the medical replacing device may further comprise an uncovering mechanism which, when activated, separates the protective element from the opening of the compartment so as to expose the opening, wherein the uncovering mechanism is configured to be activated by a changing of the medical replacing device from said first alignment position to said second alignment position. Hereby, the replacement is facilitated as one action (the action of removing the protective element) may be executed automatically as a consequence of another action (the action of changing alignment position). However, as explained above, in at least some exemplary embodiments the protective element does not necessarily need to be removed, but may instead be pierced, ruptured or cut, etc. Furthermore, in other exemplary embodiments, the removal of the protective element may be performed manually, while the action of changing alignment position may be triggered automatically as a consequence of the removing part having become actuated.

As mentioned previously it is conceivable to, in at least some exemplary embodiments, use a release film as a protective element, which can be displaced to cause the opening to become exposed so that the unused port-engaging attachment may be discharged through the exposed opening. For instance, in at least some exemplary embodiments, the medical replacing device may comprise a casing in which the receiving part of the first portion and the compartment of the second portion are provided, wherein the release film is attached to a gripping tab extending beyond an outer periphery of the casing to allow the gripping tab to be pulled for displacing the connected release film. Furthermore, in at least some exemplary embodiments, the release film and the gripping tab may together form an L-shape which extends from said opening of the compartment, laterally of and beyond the receiving part, and further beyond the outer periphery of the casing, thereby avoiding obstructing the reception of the used port-engaging attachment into the receiving part.

As mentioned previously, the port-engaging attachments may be of different configurations. In the following some different examples will be discussed.

According to at least some exemplary embodiments, the unused port-engaging attachment may be an adapter configured to interconnect said port with a supply tube which is integrated with or operatively connectable to a liquid-containing container for enabling transferring of liquid from the liquid-containing container via the supply tube, the adapter, the port, the medical tube and into the human or animal body. Thus, a medical replacing device having such an adapter stored in the compartment of the second portion of the medical replacing device, may be used when preparing for transfer of liquid into the body. In most scenarios, there will be a used port-engaging attachment, typically in the form of a lid, connected to the port for preventing fluid exchange with the port. The medical replacing device can thus be used to first remove the used port-engaging attachment by actuating the removing part in the first portion, and then the adapter (i.e. the unused port-engaging attachment) can be discharged from the compartment of the second portion upon actuation of the discharging part so as to connect the adapter to the port. The supply tube may suitably be connected to the adapter before discharging it from its stored position in compartment, however, it is also conceivable to connect the supply tube after the discharging.

According to at least some exemplary embodiments, the unused port-engaging attachment, being in the form of an adapter, may be connected to the supply tube which extends into the compartment to the unused port-engaging attachment. This is advantageous as the medical replacing device may hereby form part of a ready-to-use kit, i.e. not requiring a patient to, on his/her own, connect the liquid-containing container with its supply tube to the adapter.

According to at least some exemplary embodiments, the unused port-engaging attachment may be a lid configured to be connected to the port so as to cover the port. Thus, a medical replacing device having such a lid stored in the compartment of the second portion of the medical replacing device, may be used after liquid has been transferred into the body. In most scenarios, there will be a used port-engaging attachment, typically in the form of an adapter, connected to the port which has been used for supplying liquid to the port. The medical replacing device can thus be used to first remove the used port-engaging attachment by actuating the removing part in the first portion, and then the lid (i.e. the unused port-engaging attachment) can be discharged from the compartment of the second portion upon actuation of the discharging part so as to connect the lid to the port for protecting the port from fluid exchange.

According to at least some exemplary embodiments, the medical replacing device may comprise a securing part having a securing element which may be configured to connect to a mating securing element of the port so as to secure the medical replacing device to the port, wherein upon receipt of the used port-engaging attachment into the receiving part and while the securing part of the medical replacing device is secured to the port, the rest of the medical replacing device may be displaced relative to the securing part so as to align the second portion of the medical replacing device with the port, thereby enabling the unused port-engaging attachment to become discharged onto the port. By having a securing part which secures the medical replacing device to the port, a robust replacing procedure is achievable, mitigating the risk of inadvertent misplacements.

According to a second aspect of this disclosure, there is provided an actuating device configured to receive the medical replacing device of the first aspect, including any exemplary embodiment thereof. The actuating device may be configured to receive the medical replacing device, such as in a housing of the actuating device.

According to some exemplary embodiment of the actuating device of the second aspect, the actuating device may comprise a housing defining an internal chamber into which the medical replacing device is receivable,
wherein the housing comprises a bottom wall portion having an aperture, wherein alignment of the first portion of the received medical replacing device with the aperture enables a used port-engaging attachment to be moved by the removing part through the aperture and into the receiving part of the first portion,
wherein alignment of the second portion of the medical replacing device with the aperture, and upon displacement of the release film to expose said opening of the compartment, enables the unused port-engaging attachment to be discharged from the compartment through said opening and through the aperture in the bottom wall portion of the housing of the actuating device.

Thus, the actuating device may suitably be used for facilitating proper alignment of the of the medical replacing device for the removing and discharging actions.

According to at least some exemplary embodiments of the actuating device according to the second aspect, it may further comprise a trigger element having a non-triggering state and a triggering state,
wherein, when the medical replacing device is received in the internal chamber such that its first portion is aligned with the aperture, a change from the non-triggering state to the triggering state causes the trigger element to trigger the first actuator to cause the removing part to become actuated,
wherein, when the medical replacing device is received in the internal chamber of the actuating device such that its second portion is aligned with the aperture, a change from the non-triggering state to the triggering state causes the trigger element to trigger the second actuator to cause the discharging part to become actuated.

Although it may be conceivable to provide separate trigger elements associated with the first and second actuators, respectively, configuring one and the same trigger element to sequentially trigger both actuators reduces the risk of confusion by the patient, for example not knowing which trigger element to use at a certain instance.

According to a third aspect of this disclosure there is provided a medical replacing system, comprising:
- the medical replacing device according to the first aspect, including any exemplary embodiment thereof,
- a port configured to be connected to a medical tube that extends inside a human or animal body, wherein the port comprises a first securing element, and
- an actuating device comprising a protective housing into which the medical replacing device is receivable or is received, the actuating device being configured to control the first actuator and the second actuator of the medical replacing device, wherein the actuating device comprises a second securing element which is configured to connect with the first securing element so as to secure the actuating device to the port,
wherein, upon receipt of the used port-engaging attachment into the receiving part and while the actuating device is secured to the port, the medical replacing device is configured to be displaced relative to the protective housing so as to align the second portion of the medical replacing device with the port, thereby enabling the unused port-engaging attachment to become discharged onto said port.

By having an actuating device which can be secured to the port by cooperating securing elements, the alignment of the first portion with the port and the subsequent alignment of the second portion with the port, may be facilitated. It should, however, be understood that as explained previously, such an actuating device is not mandatory, as in some exemplary embodiments the medical replacing device may be aligned with the port without using an actuating device. The cooperating securing elements may be of any suitable type which can provide temporary securing of two components. For example, it may snap fit type of elements, threaded elements, the male/female connection elements, etc.

The actuating device included in the medical replacing system of the third aspect may, in at least some exemplary embodiments, be the actuating device presented in the second aspect of this disclosure. In other exemplary embodiments it may be a different actuating device.

In at least some exemplary embodiments the actuating device may be a separate device configured to receive the medical replacing device. In such case, after having replaced the attachment, the medical replacing device may be discarded, and the actuating device may for the next occasion receive another medical replacing device. Thus, in at least some exemplary embodiments the actuating device may be reusable, while the medical replacing device may be a single-use device. From the above it can also be understood that, in at least some exemplary embodiments, the medical replacing device may be configured as a cassette which is insertable into an actuating device and then retractable from the actuating device.

In at least some exemplary embodiments the actuating device may instead be provided with the medical replacing device already received therein. Thus, the medical replacing device may in such case be provided as being integrated with the actuating device. Furthermore, in such case, the medical replacing device and the actuating device may be single-use devices.

According to at least some exemplary embodiments of the medical replacing system of the third aspect, the actuating device may comprise a trigger element having a non-triggering state and a triggering state,
wherein, when the first portion is aligned with the port, a change from the non-triggering state to the triggering state causes the trigger element to trigger the first actuator to cause the removing part to become actuated,
wherein, when the medical replacing device has been displaced such that its second portion is aligned with the port, a change from the non-triggering state to the triggering state causes the trigger element to trigger the second actuator to cause the discharging part to become actuated.

Analogously to what has been discussed for the actuating device of the second aspect, also in this medical replacing system of the third aspect it may be beneficial to have a common trigger element for triggering the first actuator and subsequently the second actuator, thereby facilitating the handling for the patient/care recipient.

A trigger element as discussed above in relation to the second and third aspects, may be configured in various ways. In at least some exemplary embodiments, it may be configured as a lever, handle, knob, etc. which may be pressed, rotated or otherwise caused to exert a force on the first actuator and then on the second actuator. In some exemplary embodiment, the trigger element may be configured to be actively moved from either one of its states to its other state. However, in at least some exemplary embodiments, the trigger element may suitably be biased towards its non-triggering state, for example by means of a spring element. Hereby, after a person has maneuvered the trigger element to change from the non-triggering state to the triggering state, the spring element, or other type of biasing element, may automatically urge the trigger element to return to its non-triggering state.

In at least some exemplary embodiments, the medical replacing device may advantageously comprise a first latching part which it can cooperate with a second latching part of the actuating device so as to latch the medical replacing device in a first alignment position. Hereby, inadvertent relative motion of the medical replacing device may be counteracted. Thus, the first alignment position may suitably be maintained until the used port-engaging attachment has been removed and placed in the receiving part in the first portion of the medical replacing device. Suitably, such latching may be combined with the previously discussed securing of the actuating device to the port, thus enabling a reliable alignment to be achieved between the first portion and the port. The latching between the medical replacing device and the actuating device may, in at least some exemplary embodiment, be automatically unlocked upon removal of the used port-engaging attachment. This is at least partly reflected in the following example.

Thus, according to at least some exemplary embodiments of the medical replacing system of the third aspect, the medical replacing device may comprise a first latching part,
wherein the actuating device comprises a second latching part,
wherein the medical replacing device is insertable into, or pre-inserted in, the actuating device in a first alignment position in which the first and second latching parts cooperate to latch the medical replacing device, wherein in said first alignment position, while the actuating device is secured to the port, the first portion is aligned with the port, thereby enabling the used port-engaging attachment to become removed from the port,
wherein actuation of the removing part causes unlatching of the medical replacing device, allowing it to be displaced to a second alignment position in which the second portion is aligned with the port.

Hereby, a reliable alignment is achievable between the first portion and the port, as well as between the second portion and the port, without requiring much effort from a person. The unlatching may, in some exemplary embodiments, also be followed by an automatic displacement of the medical replacing device to the second alignment position. In other exemplary embodiments, the unlatching may, be followed by a manual displacement of the medical replacing device to the second alignment position.

It should furthermore be noted that, in at least some exemplary embodiments, the above described first and second latching parts, or other latching parts, may cooperate to latch the medical replacing device in the second alignment position. In such case, in particular if the actuating device is a reusable actuation device, then the actuation of the discharging part may suitably cause unlatching of the medical replacing device, allowing it to be displaced from the actuation device.

As mentioned previously, a protective element covers the opening of the compartment in which the unused port-engaging attachment is stored. Such a protective element may suitably, in at least some exemplary embodiments, have an integrated or connected gripping tab, or any other suitable gripping element, which may protrude from the actuating device when the medical replacing device is received by the actuating device. In such case, the gripping tab may suitably be pulled to cause the protective element to be removed from the opening. In exemplary embodiments in which the protective element is in the form of a release film, it would be conceivable to let the pulling of such a gripping tab initially bring along the medical replacing device in the pulling motion, so as to move the medical replacing device to the second alignment position where the medical replacing device becomes latched to the actuating device so that further pulling of the gripping tab causes the release film to be pulled off the opening to allow the unused port-engaging attachment to be discharged through the opening.

According to at least some exemplary embodiments of the medical replacing system of the third aspect, actuation of the discharging part may cause the first securing element and the second securing element to become disconnected, thereby enabling removal of the actuating device from the port. Hereby, premature removal may be avoided. Thus, the risk of the port becoming accidentally exposed can be reduced. In other words, it may be ensured that the unused port-engaging attachment becomes connected to the port before the actuating device and the medical replacing device are removed from the port. A clean and reliable replacement procedure is hereby enabled. Therefore, in practice, in connection with the attachment replacement procedure, the port may constantly be covered by at least one item. First, the used port-engaging attachment covers the port. When it is time to carry out the replacement procedure, the actuating device is secured to the port and at least the first portion with its receiving part may cover the port. Next, the second portion may cover the port, and then the unused port-engaging attachment may be discharged so that it covers the port, after which the actuating device may be disconnected from the port.

According to at least some exemplary embodiments of the medical replacing system of the third aspect, the port may have a normally closed state preventing fluid to enter or exit the medical tube through the port, and a temporary open state in which the fluid is allowed to enter or exit the medical tube through the port,
wherein attaching to the port the unused port-engaging attachment, being in the form of said adapter, causes the port to change from the normally closed state to the temporary open state, and wherein subsequent removal of the said adapter from the port causes the port to change from the temporary open state to the normally closed state.

Hereby, inadvertent fluid exchange into or out from the port may be avoided. The port may function as a valve which opens when the adapter is connected to the port and then closes when the adapter is withdrawn from the port. The port may, for instance, be in the form of a spring-biased check valve which keeps the port in said normally closed state. The adapter may, for instance, have a projecting hollow snout or nozzle which, when the adapter is connected to the port, counteracts the spring force and opens the port to allow liquid to pass through the hollow snout and through the port. Other common coupling configurations are, of course, also conceivable.

According to at least some exemplary embodiments of the medical replacing system of the third aspect, the medical replacing device is a first medical replacing device, wherein said unused port-engaging attachment is an adapter configured to interconnect the port and a supply tube which is integrated with or operatively connectable to a liquid-containing container for enabling transferring of liquid from the liquid-containing container via the supply tube, the adapter, the port, the medical tube and into the human or animal body,
wherein the medical replacing system further comprises a second medical replacing device, wherein the unused port-engaging attachment in the second medical replacing device is a lid configured to be connected to the port so as to cover the port.

As explained previously in this disclosure, the patient/care recipient may suitably be provided with a stash of medical replacing devices at home. In this example, he/she may have a stash of first medical replacing devices and a stash of second medical replacing devices. A first medical replacing device can thus be selected for connecting the adapter and initiating the supply of liquid into the body. When the liquid has been supplied into the body, a second medical replacing device may be used to provide a lid onto the port.

According to at least some exemplary embodiments of the medical replacing system of the third aspect, said unused port-engaging attachment is an adapter configured to interconnect the port and a supply tube which is integrated with or operatively connectable to a liquid-containing container for enabling transferring of liquid from the liquid-containing container via the supply tube, the adapter, the port, the medical tube and into the human or animal body, wherein the medical replacing device further comprises a third portion comprising a third actuator, an additional discharging part, an additional compartment having an additional opening, and an unused port-engaging attachment in the form of a lid stored in the compartment and arranged to be discharged through the additional opening by the additional discharging part.

This may be advantageous, as there is no need to switch to another medical replacing device after the liquid has been supplied into the body. Instead, the same medical replacing device may also be used for connecting a lid onto the port. It should be understood that this may be realized in various different ways. For instance, in some exemplary embodiments, the second portion of the medical replacing device may, in addition to the discharging part, also be equipped with an additional removing part. Thus, when the second portion is aligned with the port, the adapter may first be connected to the port for allowing liquid to be supplied into the body, and then when the supply process is completed, the adapter may be removed by said additional removing part of the second portion. In other exemplary embodiments, there may be a fourth portion having an additional removing part and an additional receiving part. Thus, after the supplying of liquid has been completed, the fourth portion may be aligned for removing and receiving the adapter from the port, and then the third portion may be aligned for connecting the lid to the port. This is reflected in at least one exemplary embodiment, according to which the medical replacing device further comprises a fourth portion comprising a fourth actuator, an additional removing part and an additional receiving part, wherein the fourth actuator is configured to cause the additional removing part to become actuated so as to enable removal of said adapter from said port and into said additional receiving part prior to said lid being discharged by said additional discharging part.

From the above, it should be understood that the medical replacing device of the first aspect (or as part of the medical replacing system of the third aspect) may be provided with more than two portions. In particular, there may be more than one compartment for storing an unused port-engaging attachment. As understood from above, there may, for example, be one compartment for storing an unused port-engaging attachment in the form of an adapter and another compartment for storing an unused port-engaging attachment in the form of a lid. The respective opening of these compartments may, in some exemplary embodiments, be covered by a common protective element, whereas in other exemplary embodiments each opening is covered by an individual protective element.

According to at least some exemplary embodiments of the medical replacing system of the third aspect, the medical replacing system may further comprises a port holder having a skin-facing side and an oppositely facing side, wherein the skin-facing side is provided with adhesive for enabling the port holder to be adhered to the skin of the human or animal body, wherein the oppositely facing side is provided with a first securing portion,
wherein the port is provided with a second securing portion which is configured to mate with the first securing portion so as to secure the port to the oppositely facing side of the port holder. This may be advantageous as the port holder may suitably be removed from the skin regularly for cleaning that area of the skin. The port holder may, suitably, be regularly replaced by another port holder, to keep the skin in good condition. For instance, the port holder may be replaced about once a week. The actual port may suitably also be replaced, however, less regularly. Medical personnel may, for instance, assist in replacing the port a few times a year. The port holder may for instance be configured as a frame into which the port may be connected, such as through a snap fit, or by any other suitably connection.

According to a fourth aspect of this disclosure, there is provided a medical container system, comprising:
- the medical replacing device according to the first aspect (including any exemplary embodiment thereof),
- a liquid-containing container, and
- a supply tube,
wherein the supply tube has an inlet end connected to the liquid-containing container, and an outlet end connected to the unused port-engaging attachment, wherein the unused port-engaging attachment is an adapter which upon connection to said port is configured to enable transferring of liquid from the liquid-containing container to said port. Hereby, a ready-to-use system is provided, thus relieving the patient/care recipient from the potentially perceived hassle of connecting the supply tube to the adapter.

The medical container system of the fourth aspect may, in at least some exemplary embodiments, be used as a stand-alone system which does not require the medical replacing device to be received by an actuating device. In other exemplary embodiments, however, the medical replacing device may suitably be arranged to be received by an actuating device, analogously to what has been discussed previously with respect to the second and third aspects of this disclosure.

Similarly, the medical replacing device of the first aspect may, in at least at least some exemplary embodiments, be used as a stand-alone device which is not required to be received by an actuating device. In other exemplary embodiments, however, the medical replacing device may suitably be arranged to be received by an actuating device, analogously to what has been discussed previously with respect to the second and third aspects of this disclosure.

The above-mentioned exemplary embodiments of a stand-alone system of the fourth aspect and a stand-alone device of the first aspect, may be conveniently be realized by providing the first and second actuators readily accessible, so as to allow the patient to manually interact with those actuators (such as be pressing or turning the actuators). Thus, no separate trigger element is needed. Furthermore, the medical replacing device may, if desired, be provided with securing elements for securing the medical replacing device in the previously discussed first and/or second alignment positions.

According to a fifth aspect there is provided a medical port system comprising the medical replacing device according to the first aspect (including any exemplary embodiment thereof) and a port connectable or connected to a medical tube that extends inside a human or animal body. The port may have any of the features discussed herein.

Generally, all terms used in the claims are to be interpreted according to their ordinary meaning in the technical field, unless explicitly defined otherwise herein. All references to "a/an/the element, portion, part, component, means, step, etc." are to be interpreted openly as referring to at least one instance of the element, portion, part, component, means, step, etc., unless explicitly stated otherwise. Further features of, and advantages with, the present inventive concept will become apparent when studying the appended claims and the following description. The skilled person realizes that different features of the present inventive concept may be combined to create embodiments other than those described in the following, without departing from the scope of the present inventive concept.

### BRIEF DESCRIPTION OF THE DRAWINGS

Figs. 1a-1c illustrate very schematically a medical replacing device in accordance with at least some exemplary embodiments of this disclosure, and a use thereof.
Fig. 2 illustrates schematically some components with which the medical replacing device of this disclosure may be used.
Fig. 3 illustrates schematically a medical container system which comprises a medical replacing device, in accordance with at least some exemplary embodiment of this disclosure.
Fig. 4 illustrates a medical replacing system in accordance with at least some exemplary embodiments of this disclosure.
Fig. 5 illustrates a cross-sectional perspective view of an actuating device that has received a medical replacing device, in accordance with at least some exemplary embodiments of this disclosure.
Fig. 6 illustrates a cross-sectional lateral view of the actuating device and medical replacing device shown in Fig. 5.
Fig. 7 illustrates a latching mechanism that may be provided for latching a medical replacing device to an actuating device, in accordance with at least some exemplary embodiments of this disclosure.
Figs. 8a-8b illustrate a securing mechanism that may be provided for securing an actuating device or a medical replacing device to a port, in accordance with at least some exemplary embodiments of this disclosure.
Figs. 9a-9b illustrate a port and a port holder, in accordance with at least some exemplary embodiments of this disclosure.
Figs. 10a-10b illustrates how the connecting of a port-engaging attachment, in the form of an adapter, to a port causes the port to open.
Figs. 11a-11b illustrates another medical replacing device, in accordance with at least some exemplary embodiments of this disclosure.
Figs. 12a-12b illustrate very schematically two other medical replacing devices, in accordance with at least some exemplary embodiments of this disclosure.

### DETAILED DESCRIPTION

The present inventive concept will now be described more fully hereinafter with reference to the accompanying drawings, in which certain aspects of the inventive concept are shown. The inventive concept may, however, be embodied in many different forms and should not be construed as limited to the embodiments and aspects set forth herein; rather, the embodiments are provided by way of example so that this disclosure will be thorough and complete, and will fully convey the scope of the inventive concept to those skilled in the art. Accordingly, it is to be understood that the present inventive concept is not limited to the embodiments described herein and illustrated in the drawings; rather, the skilled person will recognize that many changes and modifications may be made within the scope of the appended claims. Like reference numerals refer to like elements throughout the description.

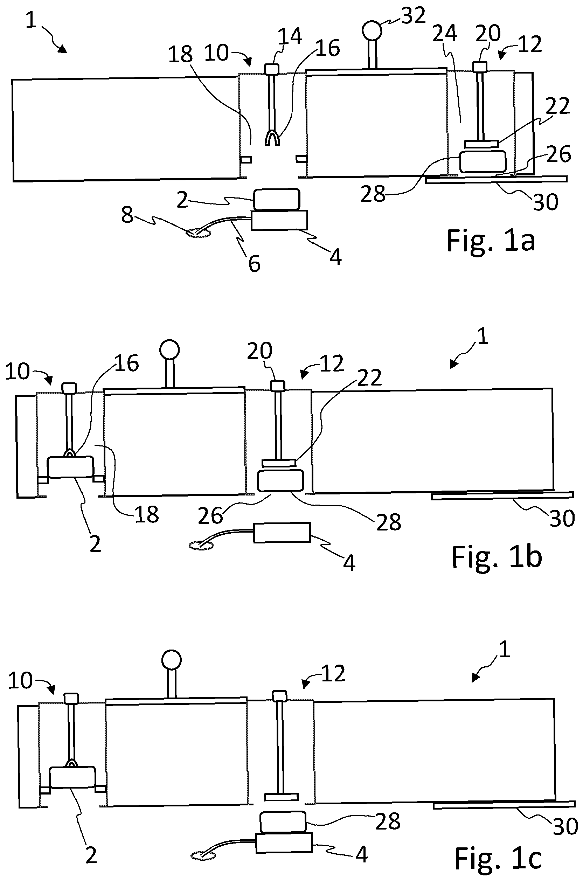

Figs. 1a illustrates very schematically a medical replacing device 1 in accordance with at least some exemplary embodiments of this disclosure. The medical replacing device 1 is configured for replacing a used port-engaging attachment 2 which is engaged with a port 4 connected to a medical tube 6 that extends inside a human or animal body. Here an entry 8 into a body is schematically illustrated and the medical tube 6 extends from the port 4 on the outside of the body and through the entry 8 and into the body. In other examples, however, the port 4 itself may be arranged to extend through the skin, thus, in which case the medical tube 6 does not extend from the outside and into the body but just extends inside the body.

Fig. 1a illustrates that the medical replacing device 1 comprises a first portion 10 and a second portion 12. The first portion 10 comprises a first actuator 14, a removing part 16 and a receiving part 18. The first actuator 14 is configured to cause the removing part 16 to become actuated for removing the used port-engaging attachment 2 from the port 4 and into the receiving part 18. The receiving part 18 may simply be any space or area of the medical replacing device 1 in which the removed used port-engaging attachment 2 can be kept.

The second portion 12 comprises a second actuator 20, a discharging part 22, a compartment 24 having an opening 26 and an unused port-engaging attachment 28 stored in the compartment 24 and arranged to be discharged through the opening 26 by the discharging part 22. A protective element 30 covers the opening 26 of the compartment 24 to counteract discharging of the unused port-engaging attachment 28 through the opening 26. It should be noted that although the protective element 30 counteracts discharging, in some examples, such counteracting may be overcome. For instance, as previously discussed the protective element 30 may in some examples be a film that may be ruptured or pierced by pressing the unused port-engaging attachment 28 against and through the film. However, in other examples, such as illustrated in Figs. 1a-1c, the protective element 30 may suitably be separable from the opening 26.

Turning now to Fig. 1b, there is illustrated that the removing part 16 of the first portion 10 has picked up the used port-engaging attachment 2 from the port 4 to keep it in the receiving part 18. Upon receipt of the used port-engaging attachment 2 into the receiving part 18, the second actuator 20 is configured to cause the discharging part 22 to become actuated for discharging the unused port-engaging attachment 28 through the opening 26 and onto the port 4. However, as explained previously in this disclosure, in at least some exemplary embodiments, the proper functioning of the second actuator 20 is not limited to or dependent on the reception of the used port-engaging attachment 2 into the receiving part 18. As mentioned above, in this example, the protective element 30 has been separated from the opening 26.

Fig. 1c illustrates the unused port-engaging attachment 28 having been discharged through the opening 26 and is now attached to the port 4.

By providing a medical replacing device 1 a person does not need to manually handle the port-engaging attachments 2, 28, neither the used, nor the un-used port-engaging attachments 2, 28. Thus, a more hygienic handling can be achieved. As has already been discussed elsewhere in this disclosure, and will be discussed in more detail later, the used port-engaging attachment 2 may be a lid and the unused port-engaging attachment 28 may be an adapter for supplying liquid from a liquid containing container to the port 4. In other examples, it may be the other way around, i.e. the used port-engaging attachment 2 may be an adapter and the unused port-engaging attachment 28 may be a lid.

In at least one aspect, the medical replacing device 1 and the port 4 may together form part of a medical port system.

Although not illustrated in the schematic drawings of Figs. 1a-1c, it should be understood that the medical replacing device 1 may suitably comprise a securing element for cooperating with a corresponding securing element of the port 4 in order to hold the medical replacing device 1 in position, during the replacing procedure. While a casing or frame of the medical replacing device 1 is kept stationary in relation to the port 4, the first and second portions 10, 12 may suitably be movable to enable proper alignment. In this example, a simple handle 32 has been illustrated to show that the first and second portions 10, 12 may be moved, so as to change from a first alignment position in Fig. 1a, to a second alignment position in Figs. 1b-1c.

In the first alignment position (Fig. 1a), the first portion 10 is aligned with the port 4, wherein activation of the first actuator 14 causes the removing part 16 to remove the used port-engaging attachment 2 from the port 4 and into the receiving part 18. In the second alignment position (Fig. 1b and 1c) the second portion 12 is aligned with the port 4. Without any port-engaging attachment connected to the port 4 (Fig. 1b), activation of the second actuator 20 causes the discharging part to discharge the unused port-engaging attachment 28 through the opening 26 and onto the port 4 (Fig. 1c).

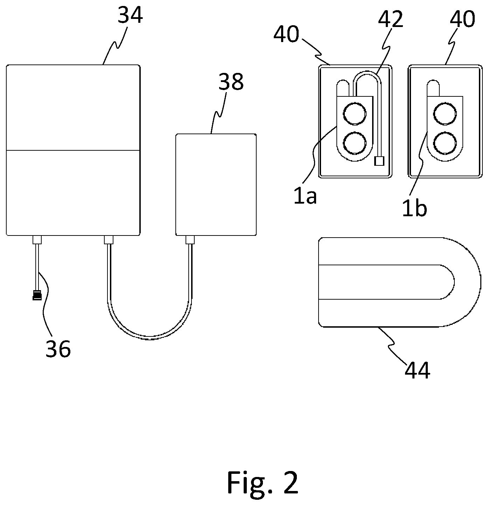

Fig. 2 illustrates schematically some components with which a medical replacing device of this disclosure may be used. A liquid-containing container 34, such as a dialysis bag (for example, a peritoneal dialysis bag) is shown in Fig. 2. A supply tube 36 is integrated with the liquid-containing container 34. In the illustrated example, there is also shown that a drainage bag 38 may be connected to the liquid-containing container 34. In other examples, the supply tube may include a Y-coupling so as to form a bifurcated arrangement, such that one branch of the supply tube extends from the liquid-containing container and the other branch extends from the drainage bag, wherein the two branches are joined to a common stem of the supply tube (including any coupling) for passing liquid to/from the patient. It should, however, be understood that the general inventive concept is not limited to these examples but may be conveniently implemented also in relation to single liquid-containing containers without drainage bags. Furthermore, the liquid-containing container 34 can be other types of containers than for dialysis, e.g. the content may include saline solutions, dextrose solutions, medications, etc. Furthermore, in some exemplary embodiments, the liquid containing container may be connected to a cycler machine (which may also be referred to as an automated dialysis machine), which is configured to pump the liquid through the supply tube to and from the patient. Thus, in such exemplary embodiments, the supply tube can be operatively connectable to the liquid containing container via the cycler machine. The general inventive concept may be implemented also for use in connection with an arrangement containing such a cycler machine.

Fig. 2 illustrates two medical replacing devices 1a, 1b, namely a first medical replacing device 1a and a second medical replacing device 1b, each stored in a respective package 40. The first medical replacing device 1a may comprise an unused port-engaging attachment in the form of an adapter (not shown) to which is connected an adapter tube 42. When it is time to supply the liquid from the liquid-containing container 34 into a human or animal body, the first medical replacing device 1a may be removed from its package 40 and the supply tube 36 may be connected to the adapter tube 42. In other exemplary embodiments, the adapter tube 42 may be omitted, and the supply tube 36 may be connected directly to the adapter. In further exemplary embodiments, the adapter tube 42 may be pre-connected to, or be connectable to, a manifold for use in a cycler machine, such as the example mentioned above. In such case, the supply tube may be considered to form part of the manifold. In still further exemplary embodiments, the adapter may be directly connectable to, or even be pre-connected to, such a manifold. In either case, the manifold may be configured to be docked to a cycler machine for pumping liquid from the liquid-containing container to the adapter.

Upon removal of the used port-engaging attachment from the port by means of the first medical replacing device 1s, and subsequent discharging of the adapter onto the port, the adapter interconnects the port and the supply tube 36 for enabling transferring of liquid from the liquid-containing container 34 via the supply tube 36, the adapter, the port, the medical tube and into the human or animal body. The second medical replacing device 1b may comprise an unused port-engaging attachment in the form of a lid (not shown). After completion of the supplying of liquid from the liquid-containing container 34, the second medical replacing device 1b may be taken out from its package 40 and used for removing the adapter from the port and replacing it with the lid which becomes discharged and attached to the port. The lid may thus stay on the port until it is again time to perform liquid supply treatment. A patient or care recipient may suitably be given a large batch of first medical replacing devices 1a and a similar large batch of second medical replacing devices 1b, to allow for multiple liquid supply treatments over time.

Fig. 2 also illustrates schematically an actuating device 44. As will be shown in other drawing figures, in at least some exemplary embodiments, medical replacing devices (such as the medical replacing devices 1a, 1b in Fig. 2) may suitably be operated by means of such an actuating device 44. It should, however, be understood that the general inventive concept does not require an actuating device 44 to be used, but allows, in at least some exemplary embodiments, the medical replacing device (such as medical replacing devices 1a, 1b) to be operated without an actuating device 44.

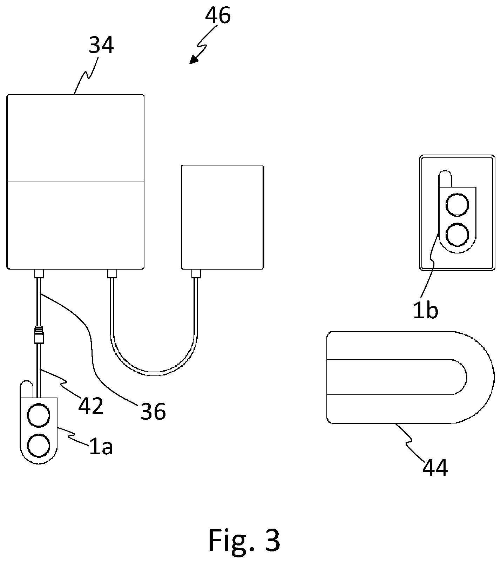

Fig. 3 illustrates schematically a medical container system 46 which comprises a medical replacing device 1a, in accordance with at least some exemplary embodiment of this disclosure. In particular, Fig. 3 illustrates a medical container system 46 which comprises a liquid containing container 34 and a supply tube 36. The supply tube 36 has an inlet end connected to the liquid containing container 34. The medical container system 46 further comprises a medical replacing device 1a which, in accordance with this disclosure has a second portion with a compartment in which an unused port-engaging attachment is stored.

The port-engaging attachment is in the form of an adapter. The supply tube 36 is connected to the adapter. Thus, upon connection of the adapter to the port, liquid may be transferred from the liquid-containing container 34 to the port. It can therefore be understood that rather than having a separate medical replacing device having a stored adapter (such as the first medical replacing device 1a in the example of Fig. 2), the medical container system 46 of Fig. 3 may suitably be delivered with the medical replacing device 1a already connected, whereby a handling step can be avoided (i.e. the step of connecting the supply tube 36 to the adapter or adapter tube 42). Thus, a person or care recipient may suitably be given a batch of such medical container systems 46, and in addition may suitably be given a corresponding batch of medical replacing devices 1b in which an unused port-engaging attachment in the form of a lid is stored (similarly to the second medical replacing 1b device in Fig. 2). Again, in Fig. 3 an optional actuating device 44 is schematically indicated.

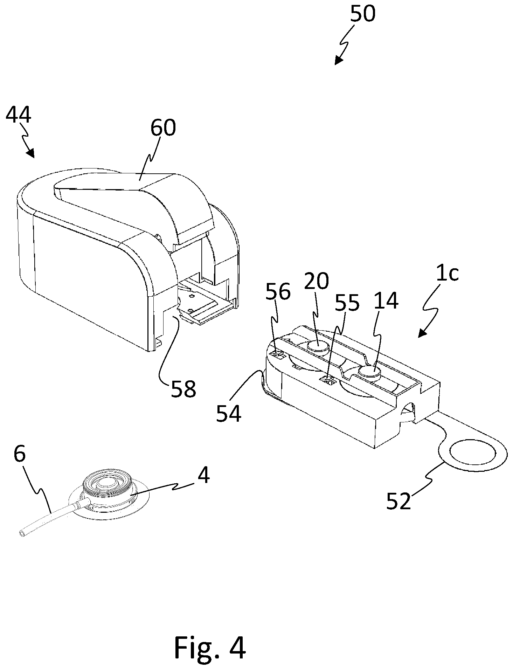

Fig. 4 illustrates a medical replacing system 50 in accordance with at least some exemplary embodiments of this disclosure. The medical replacing system 50 comprises a medical replacing device 1c, a port 4, and an actuating device 44.

The port 4 is configured to be connected to a medical tube 6 that extends inside a human or animal body. The port 4 may comprise a first securing element, the function of which will be described in more detail further below. However, briefly, such a first securing element may be formed by any suitable, catch, grove, projection, or simply by the circumferential rim or peripheral surface of the port 4.

For the medical replacing device 1c, in this illustrated example, the first actuator 14 and the second actuator 20 are visible. In this example, the first and second actuators 14, 20 have a button or mushroom-shaped configuration. However, other shapes are readily conceivable. A gripping part 52 of the medical replacing device 1c is visible. In some exemplary embodiments, pulling of the gripping part 52 causes the medical replacing device 1c, when docked to the actuating device 44, to be moved from a first alignment position to a second alignment position. In some exemplary embodiments, the gripping part 52 may be connected to or form part of the protective element 54, allowing a user to pull the protective element 54 relative to the rest of the medical replacing device 1c. In some exemplary embodiments, pulling of the gripping part 52 may first cause the medical replacing device 1c to move from the first alignment position to the second alignment position, and then further pulling (with the medical replacing device 1c being latched in the second alignment position) may peel off the protective element 54 from the opening to expose the opening. The medical replacing device 1c is also provided with latches 55, 56 for enabling latching of the medical replacing device 1c to the actuating device 44. The function of these latches 55, 56 will be described in more detail further below.

The actuating device 44 is in this example illustrated as having an opening 58 through which the medical replacing device 1c is insertable into an internal chamber of the actuating device 44. However, in other examples the actuating device 44 may be configured as a more open structure, for example without an internal chamber for receiving the medical replacing device 1c. The actuating device 44 has a trigger element 60, which in this example is illustrated as a key or lever. The trigger element 60 is illustrated in a non-triggering state. A user can press the trigger element 60 to move it to a triggering state. In other examples, the trigger element 60 may be configured differently, such as in the form of a button, knob, etc. Furthermore, in other examples, changing from the non-triggering state to the triggering state may be achieved in other ways, such as by a turning or rotating action instead of a pressing action.

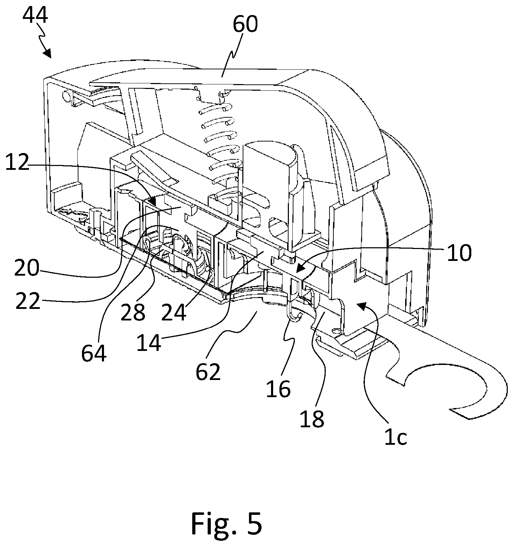

Fig. 5 illustrates a cross-sectional perspective view of an actuating device 44 that has received a medical replacing device 1c, in accordance with at least some exemplary embodiments of this disclosure. The actuating device 44 and the medical replacing device 1c may, for example, correspond to the actuating device 44 and medical replacing device 1c illustrated in Fig. 4.

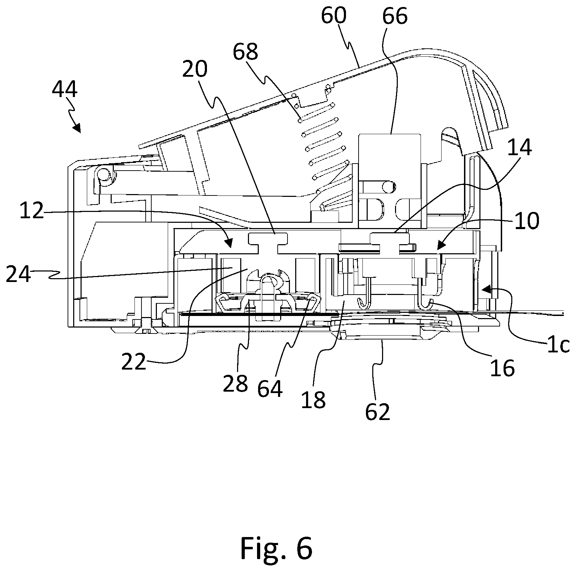

Fig. 6 illustrates a cross-sectional lateral view of the actuating device 44 and the medical replacing device 1c shown in Fig. 5. As illustrated in Figs. 5 and 6, the medical replacing device 1c has been received by the actuating device 44. The first portion 10 and the second portion 12 of the medical replacing device 1c are visible in Figs. 5 and 6. As can be seen in Figs. 5 and 6, the first portion 10 is aligned with an aperture 62 of the actuating device 44. Also visible, at the first portion 10, are the first actuator 14, the removing part 16 and the receiving part 18. The receiving part 18 is the open volume into which a used port-engaging attachment is intended to be received. The removing part 16 is in this example represented by hooks for gripping the used port-engaging attachment and bringing it to the receiving part 18. With respect to the second portion 12, the second actuator 20, the discharging part 22, the compartment 24 and the unused port-engaging attachment 28 are visible. With respect to the unused port-engaging attachment 28, its circumferential rim 64 is folded inwardly, forming a Cor U-shaped cross-section. The used port-engaging attachment to be removed, may suitably also have such a circumferential rim forming a C- or U-shaped cross-section, allowing the hooks of the removing part 16 to get a grip.

The trigger element 60 of the actuating device 44 is shown in its non-triggering state. Upon pressing the trigger element 60 downwards to its triggering state, it will exert a downwardly directed force on a force transferring element 66, which will transfer the force to the first actuator 14, which in turn causes the removing part 16 to become actuated for removing the used port-engaging attachment from the port and into the receiving part 18. The actuating device 44 comprises a spring 68, by means of which the trigger element 60 is spring biased towards its non-triggering state. Thus, upon releasing the trigger element 60 in its triggering state, the spring 68 will urge the trigger element 60 to return to its non-triggering state.

When the second portion 12 has been aligned with the aperture 62 of the actuating device 44, the trigger element 60 may once again be pressed, this time to discharge the unused port-engaging attachment 28 onto the port.

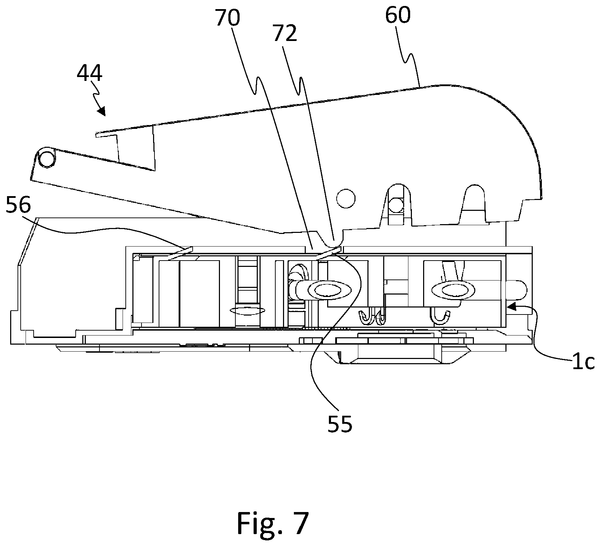

Fig. 7 illustrates a latching mechanism that may be provided for latching a medical replacing device 1c to an actuating device 44, in accordance with at least some exemplary embodiments of this disclosure. As previously illustrated in Fig. 4 the medical replacing device 1c may be provided with latches 55, 56 for latching the medical replacing device 1c to the actuating device 44. In Fig. 7, it can be seen that upon insertion of the medical replacing device 1c into the actuating device 44 (insertion direction is from right to left in the drawing figure), a first latching part 55 of the medical replacing device 1c has snapped into a second latching part 70 in the form of a slot or the like of the actuating device 44. With the first latching part 55 being snapped into the second latching part 70, a reverse movement of the medical replacing device (from left to right in the drawing figure) is counteracted.

A bulging part 72 is operatively connected to the trigger element 60. When the trigger element 60 is pressed from its non-triggering state to its triggering state, it will bring along the bulging part 72 in its motion. The bulging part 72 will, in turn, press down first latching part 55 causing it to become bent (such as beyond its yield strength) so that it cannot snap back into the second latching part 70. Hereby, if the actuating device 44 is secured to the port, then the medical replacing device 1c may be kept latched in a first alignment position until the used port-engaging attachment has been removed. Upon unlatching the first and second latching parts 55, 70 from each other, the medical replacing device 1c may be changed to its second alignment position. As shown in Fig. 7, there may be a further latching part 56 of the medical replacing device 1c which is configured to become engaged with the latching part 70 of the actuating device 44 to maintain the medical replacing device 1c in the second alignment position until the unused port-engaging attachment has been discharged onto the port.

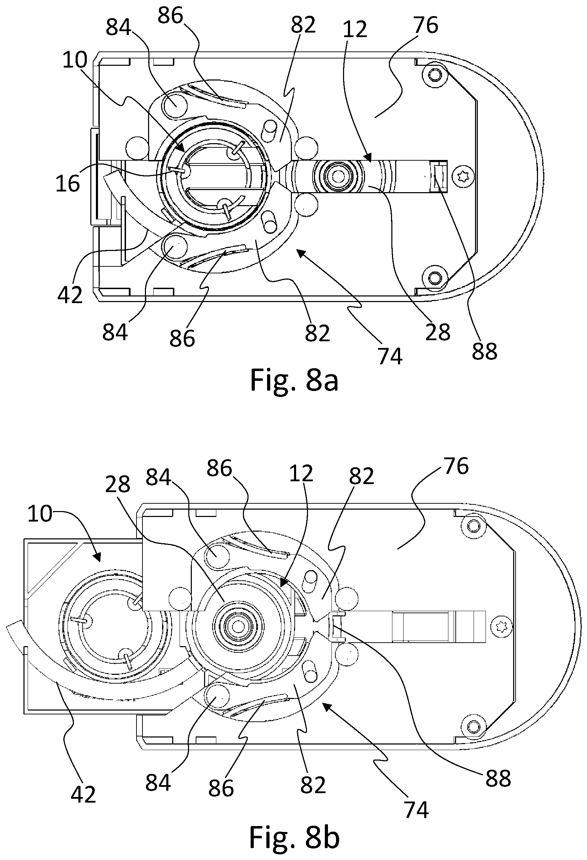

Figs. 8a-8b illustrate a securing mechanism 74 that may be provided for securing an actuating device or a medical replacing device to a port, in accordance with at least some exemplary embodiments of this disclosure. For instance, Figs. 8a-8b may illustrate an actuating device such as previously exemplified in Figs. 4-7, in which case the actuating device is seen from below, i.e. a bottom side 76 intended to face the port. However, it should be noted that the same securing principle could also be used without an actuating device. Thus, Figs. 8a-8b could instead represent a medical replacing device having an integrated securing mechanism 74 for securing it to a port. In such case, the illustrated lower bottom side 76 could represent a securing part of the medical replacing device, and could also represent a protective element.

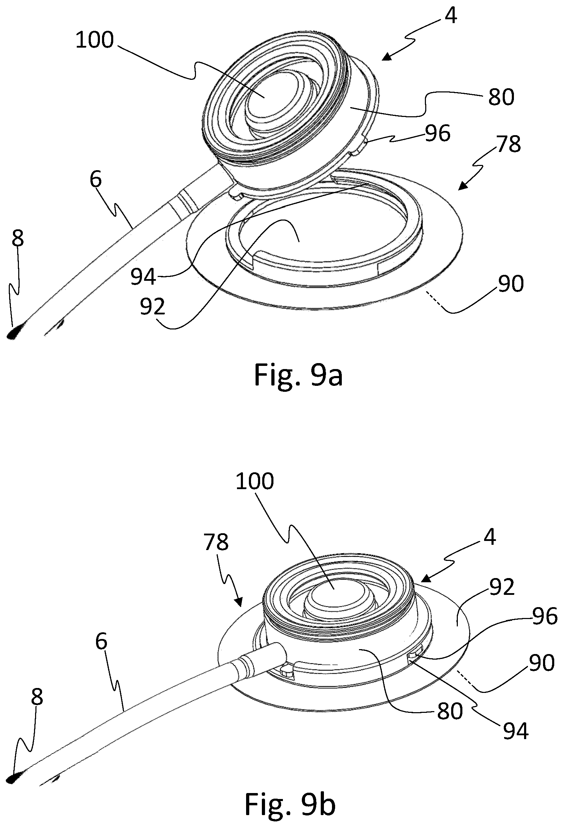

Before continuing with the description of Figs. 8a-8b, brief reference is first made to Figs. 9a-9b which illustrate a port 4 and a port holder 78, in accordance with at least some exemplary embodiments of this disclosure. The port 4 comprises a first securing element 80, which may in this example be represented by its circumferential enveloping surface.

Turning now back to Figs. 8a-8b, assuming a bottom side 76 of an actuating device is shown, the actuating device provided with a second securing element 82, herein illustrated as comprising a pair of jaws 82. The second securing element 82 may thus form part of the overall securing mechanism 74. Each jaw 82 may be pivotably connected to a pivot axle 84. Spring elements 86 urge the jaws 82 towards each other. When the actuating device is connected to the port, the port will urge the jaws 82 to separate so as to fit the port between the jaws 82, which will by means of the spring action of the spring elements 86 clamp the port. In particular, with respect to the port 4 in Figs. 9a-9b, it will be the first securing element 80 (represented by the enveloping surface) that will be clamped by the jaws 82.

Fig. 8a shows the medical replacing device in a first alignment position, the first portion 10 being visible with its removing part 16 (including the three visible hooks). The second portion 12 is also slightly visible in Fig. 8a. Furthermore, in both Figs. 8a and 8b, an unused port-engaging attachment 28 is visible in the second portion 12, the unused port-engaging attachment 28 being in the form of an adapter having an adapter tube 42 connected to it. Turning now to Fig. 8b, it shows that the medical replacing device has now been moved away from the first alignment position. Furthermore, in Fig. 8b, although the unused port-engaging attachment 28 is still shown as being present in the second portion, it can be understood that after the unused port-engaging attachment 28 is discharged, a further displacement of the medical replacement device causes a small projecting part 88 of the medical replacing device to press the jaws apart 82, thereby enabling the actuating device to become unsecured and released from the port.

In the alternatives in which Figs. 8a-8b represent a securing part of the medical replacing device (i.e. no actuating device being present or used), then same principle applies. The bottom part 76 may be a combined securing part and protective element. The securing part has said pair of jaws 82, and operate in the same way as explained above. When the securing part is secured to the port, the rest of the medical replacing device may be moved relative to the securing part so as to change from the first alignment position to the second alignment position. Similarly, the small projecting part 88 may cause the jaws 82 to open to release the securing part from the port.

Continuing with Figs. 9a-9b, the port holder 78 has a skin-facing side 90 and an oppositely facing side 92. The skin-facing side 90 may be provided with adhesive for enabling the port holder 78 to be adhered to the skin of a human or animal body. The oppositely facing side 92 is provided with a first securing portion 94, such as a track. The port 4 is provided with a second securing portion 96, such as teeth, splines, etc. It should, however, be understood that any other suitable configurations of securing portions are readily conceivable. The second securing portion 96 is configured to mate with the first securing portion 94 so as to secure the port 4 to said oppositely facing side 92 of the port holder 78, as illustrated in Fig. 9b. Also illustrated in Figs. 9a-9b is a port-engaging attachment 100 in the form of a lid, which protects the port 4 when no liquid treatment is being performed, e.g. it may protect the port from becoming dirty, becoming accidently opened, tampered with, etc. Figs. 9a-9b also illustrate a medical tube 6 extending from the port 4 into a human or animal body 8.

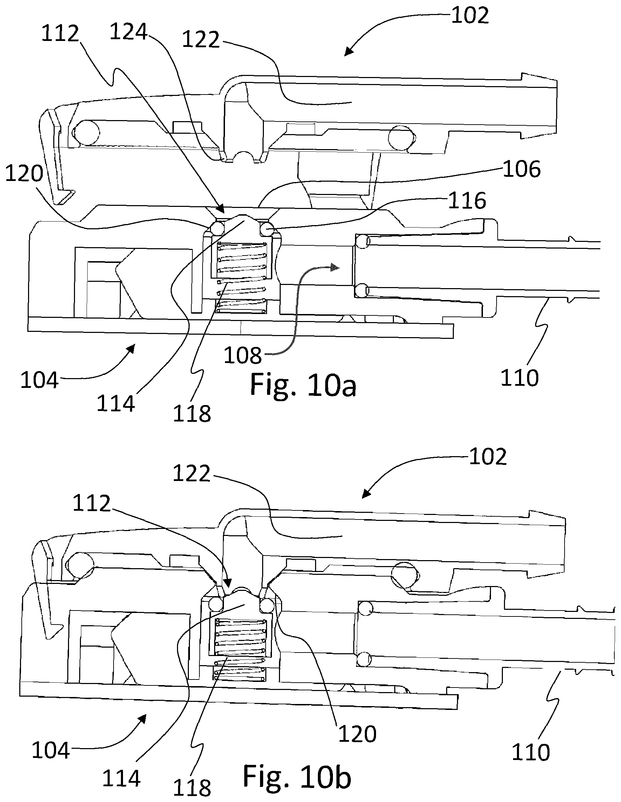

Figs. 10a-10b illustrates how the connecting of a port-engaging attachment 102, in the form of an adapter, to a port 104 causes the port 104 to open. It should be noted that this is just one exemplary embodiment, and that others are readily conceivable. Starting with Fig. 10a, the port 104 has an inlet 106 for receiving supplied liquid and an outlet 108 for passing the supplied liquid to a medical tube 110. The port 104 is provided with a spring-biased check valve 112. The check valve 112 has a valve plug 114 provided with an O-ring 116. The valve plug 114 is pressed by a spring element 118 against a valve seat 120 to seal/close the check valve 112. Hereby liquid is prevented from flowing from the inlet 106 to the connected medical tube 110, and vice versa. Fig. 10a, also illustrates that the port-engaging attachment 102 is provided with a passage 122 for guiding liquid from a supply tube connected to a liquid-containing container (such as previously discussed and illustrated). The port-engaging attachment 102 has a projecting nozzle or snout 124 which forms an outlet of the passage 122.

Turning now to Fig. 10b, the port-engaging attachment 102 has been connected to the port, 104 which has caused the snout 124 to push the valve plug 114 away from the valve seat 120, hereby enabling fluid communication to be established from the passage 122 of the port-engaging attachment 102 and through the port 104 to the medical tube 110. This is the temporary open state of the port 104. Upon later removal of the port-engaging attachment 102, the spring element 118 will return the valve plug 114 to close the check valve 112, i.e. the port 104 will be returned to its normally closed state, as in Fig. 10a. Port-engaging attachments in the form of a lid are suitably configured without a snout or any other part that would accidentally push the valve plug 114 away from the valve seat 120.

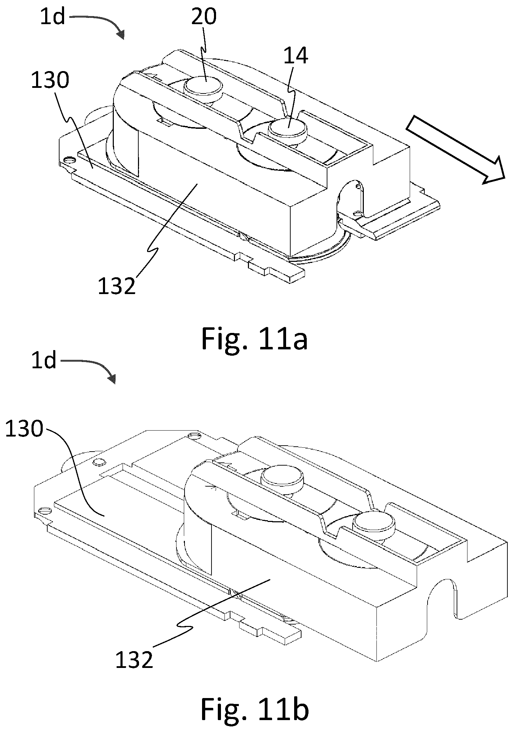

Figs. 11a-11b illustrates another medical replacing device 1d, in accordance with at least some exemplary embodiments of this disclosure. In this example no actuating device is used. Instead, the first actuator 14 and the second actuator 20 of the medical replacing device 1d may be triggered by a user pressing directly on the respective actuator 14, 20, i.e. instead of pressing a trigger element such as a key or lever of an actuating device as discussed for example in relation to Fig. 5. The medical replacing device 1d in Fig. 11a-11b comprises a securing part 130 which is connected to a main part 132 of the medical replacing device 1d. The main part 132 is provided with the first portion and the second portion (including the first and second actuators 14, 20). Although the lower side is not visible in Figs. 11a-11b, the securing part 130 may suitably have a securing element, such as the one with the pair of jaws 82 discussed in relation to Figs 8a-8b, however, other types of securing elements are also conceivable. The securing part 130 may suitably also function as a protective element which, at least initially covers the opening of the compartment of the second portion.

In Fig. 11a the medical replacing device 1d is configured in a first alignment position. With the securing part 130 secured to the port, in the first alignment position, the first portion may be aligned with the port so that a used port-engaging attachment may be removed from the port and retrieved to the receiving part (for instance, through an aligned aperture of the securing part 130). As indicated by the arrow in Fig. 11a, the main part 132 of the medical replacing device may be displaced relative to the securing part 130 to arrive a second alignment position, which is illustrated in Fig. 11b. In the second alignment position, the second portion is aligned with the port (and said aperture of the securing part 130). Now, the unused port-engaging attachment may be discharged onto the port.

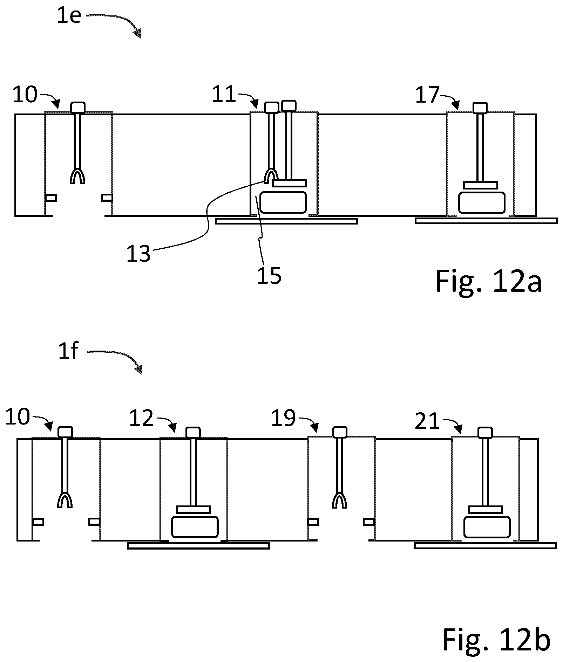

Figs. 12a-12b illustrate very schematically two other medical replacing devices 1e, 1f, in accordance with at least some exemplary embodiments of this disclosure. Starting with Fig. 12a, there is illustrated a medical replacing device 1e, having a first portion 10 which may be configured similarly to what has been previously discussed with respect to first portions 10 of other exemplary embodiments. The medical replacing device 1d also has a second portion 11. The second portion 11 may also have features that correspond to the features of second portions 12 discussed herein with respect to other exemplary embodiments. However, in this exemplary embodiment in Fig. 12a, the second portion 11 further has an additional removing part 13, and an additional receiving part 15. The port-engaging attachment in the compartment of the second portion 11 may suitably be an adapter. Upon completion of the treatment, the adapter may suitably be removed by the additional removing part 13 and returned back to the additional receiving part 15 of the second portion 11. The medical replacing device 1d further comprises a third portion 17 in which a port-engaging attachment in the form of a lid is stored. Thus, when the adapter has been discharged from and then returned to the second portion 11, the lid may be discharged onto the port from the third portion 17. In this example separate protective elements have schematically been indicated for the openings of the compartments of the second and third portion, respectively, however, in other exemplary embodiments they may share one common protective element.

Fig. 12b shows another exemplary embodiment of a medical replacing device 1f. Here there are four portions 10, 12, 19, 21. Two portions 10, 19 for removing and receiving used port-engaging attachments, and two portions 12, 21 for discharging a respective unused port-engaging attachment (e.g. one of them may have an adapter and the other may have a lid).

## Claims

1. A medical replacing device configured for replacing a used port-engaging attachment which is engaged with a port connected to a medical tube that extends inside a human or animal body, wherein the medical replacing device comprises:
- a first portion comprising a first actuator, a removing part and a receiving part, wherein the first actuator is configured to cause the removing part to become actuated for removing said used port-engaging attachment from said port and into the receiving part,
- a second portion comprising a second actuator, a discharging part, a compartment having an opening, and an unused port-engaging attachment stored in the compartment and arranged to be discharged through the opening by the discharging part,
- a protective element which covers said opening of the compartment for maintaining the unused port-engaging attachment in the compartment,
wherein the second actuator is configured to cause the discharging part to become actuated for discharging the unused port-engaging attachment through the opening and onto said port.

2. The medical replacing device according to claim 1, wherein, upon receipt of the used port-engaging attachment into the receiving part, the second actuator is configured to cause the discharging part to become actuated for discharging the unused port-engaging attachment through the opening and onto said port.

3. The medical replacing device according to any one of claims 1-2, wherein the medical replacing device is configured to be aligned with said port in two different alignment positions, wherein
- in a first alignment position the first portion is aligned with said port, wherein in said first alignment position, and with the used port-engaging attachment connected to said port, activation of the first actuator causes the removing part to remove the used port-engaging attachment from the port and into the receiving part, and
- in a second alignment position the second portion is aligned with said port, wherein, in said second alignment position, and without any port-engaging attachment connected to the port, activation of the second actuator causes the discharging part to discharge the unused port-engaging attachment through the opening and onto said port.

4. The medical replacing device according to claim 3, further comprising an uncovering mechanism which, when activated, separates the protective element from the opening of the compartment so as to expose the opening, wherein the uncovering mechanism is configured to be activated by a changing of the medical replacing device from said first alignment position to said second alignment position.

5. The medical replacing device according to any one of claims 1-4, wherein the unused port-engaging attachment is an adapter configured to interconnect said port with a supply tube which is integrated with or operatively connectable to a liquid-containing container for enabling transferring of liquid from the liquid-containing container via the supply tube, the adapter, the port, the medical tube and into the human or animal body.

6. The medical replacing device according to claim 5, wherein the unused port-engaging attachment is connected to the supply tube which extends into the compartment to the unused port-engaging attachment.

7. The medical replacing device according to any one of claims 1-4, wherein the unused port-engaging attachment is a lid configured to be connected to the port so as to cover the port.

8. A medical replacing system, comprising
- the medical replacing device according to any one of claims 1-7,
- a port configured to be connected to a medical tube that extends inside a human or animal body, wherein the port comprises a first securing element, and
- an actuating device comprising a protective housing into which the medical replacing device is receivable or is received, the actuating device being configured to control the first actuator and the second actuator of the medical replacing device, wherein the actuating device comprises a second securing element which is configured to connect with the first securing element so as to secure the actuating device to the port, wherein, upon receipt of the used port-engaging attachment into the receiving part and while the actuating device is secured to the port, the medical replacing device is configured to be displaced relative to the protective housing so as to align the second portion of the medical replacing device with the port, thereby enabling the unused port-engaging attachment to become discharged onto said port.

9. The medical replacing system according to claim 8, wherein the actuating device comprises a trigger element having a non-triggering state and a triggering state, wherein, when the first portion is aligned with the port, a change from the non-triggering state to the triggering state causes the trigger element to trigger the first actuator to cause the removing part to become actuated,
wherein, when the medical replacing device has been displaced such that its second portion is aligned with the port, a change from the non-triggering state to the triggering state causes the trigger element to trigger the second actuator to cause the discharging part to become actuated.

10. The medical replacing system according to any one of claims 8-9,
wherein the medical replacing device comprises a first latching part,
wherein the actuating device comprises a second latching part,
wherein the medical replacing device is insertable into, or pre-inserted in, the actuating device in a first alignment position in which the first and second latching parts cooperate to latch the medical replacing device, wherein in said first alignment position, while the actuating device is secured to the port, the first portion is aligned with the port, thereby enabling the used port-engaging attachment to become removed from the port, wherein actuation of the removing part causes unlatching of the medical replacing device, allowing it to be displaced to a second alignment position in which the second portion is aligned with the port.

11. The medical replacing system according to any one of claims 8-10, wherein actuation of the discharging part causes the first securing element and the second securing element to become disconnected, thereby enabling removal of the actuating device from the port.

12. The medical replacing system according to any one of claims 8-11,
wherein the medical replacing device is a first medical replacing device, wherein said unused port-engaging attachment is an adapter configured to interconnect the port and a supply tube which is integrated with or operatively connectable to a liquid-containing container for enabling transferring of liquid from the liquid-containing container via the supply tube, the adapter, the port, the medical tube and into the human or animal body, wherein the medical replacing system further comprises a second medical replacing device, wherein the unused port-engaging attachment in the second medical replacing device is a lid configured to be connected to the port so as to cover the port.

13. The medical replacing system according to any one of claims 8-11
wherein said unused port-engaging attachment is an adapter configured to interconnect the port and a supply tube which is integrated with or operatively connectable to a liquid-containing container for enabling transferring of liquid from the liquid-containing container via the supply tube, the adapter, the port, the medical tube and into the human or animal body,
wherein the medical replacing device further comprises a third portion comprising a third actuator, an additional discharging part, an additional compartment having an additional opening, and an unused port-engaging attachment in the form of a lid stored in the compartment and arranged to be discharged through the additional opening by the additional discharging part,
wherein the medical replacing device, optionally, further comprises a fourth portion comprising a fourth actuator, an additional removing part and an additional receiving part, wherein the fourth actuator is configured to cause the additional removing part to become actuated so as to enable removal of said adapter from said port and into said additional receiving part prior to said lid being discharged by said additional discharging part.

14. The medical replacing system according to any one of claims 11-12, wherein the medical replacing system further comprises a port holder having a skin-facing side and an oppositely facing side, wherein the skin-facing side is provided with adhesive for enabling the port holder to be adhered to the skin of the human or animal body, wherein the oppositely facing side is provided with a first securing portion,
wherein the port is provided with a second securing portion which is configured to mate with the first securing portion so as to secure the port to the oppositely facing side of the port holder.

15. A medical container system, comprising:
- the medical replacing device according to any one of claims 1-7,
- a liquid-containing container, and
- a supply tube,
wherein the supply tube has an inlet end connected to the liquid-containing container, and an outlet end connected to the unused port-engaging attachment, wherein the unused port-engaging attachment is an adapter which upon connection to said port is configured to enable transferring of liquid from the liquid-containing container to said port.
